# EUROPÄISCHE PATENTSCHRIFT

(11) **EP 3 426 252 B1**
(45) Veröffentlichungstag und Bekanntmachung des Hinweises auf die Patenterteilung: **02.07.2025**
(21) Anmeldenummer: 17716131.2
(22) Anmeldetag: 13.03.2017
(51) Int. Cl.: A61K 31/505, A61P 11/00, A61K 9/127, A61K 9/51, A61P 1/02, A61P 35/02, A61P 35/00

(54) **ZUSAMMENSETZUNG ZUR FÖRDERUNG DER AKTIVITÄT VON SIRTUINEN**
COMPOSITION FOR STIMULATING SIRTUIN ACTIVITY
COMPOSITION SERVANT À STIMULER L'ACTIVITÉ DE SIRTUINES

(30) Priorität: 11.03.2016 DE 102016104470
(43) Veröffentlichungstag der Anmeldung: 16.01.2019
(73) Patentinhaber: Bitop AG, Witten 58453 (DE)
(72) Erfinder: BILSTEIN, Andreas, 50129 Bergheim (DE); KRUTMANN, Jean, 40237 Düsseldorf (DE); UNFRIED, Klaus, 40225 Düsseldorf (DE)
(74) Vertreter: Heide, Anna Katharina
(86) Internationale Anmeldenummer: PCT/EP2017/055843
(87) Internationale Veröffentlichungsnummer: WO 2017/153609

(56) Entgegenhaltungen:
- EP-A2- 2 366 375
- WO-A1-2005/002556
- WO-A1-2013/034299
- WO-A1-2016/045714
- BITOP AG: "Ectoin Scientific Information", 31 March 2010
- GRAF R ET AL: "The multifunctional role of ectoine as a natural cell protectant", CLINICS IN DERMATOLOGY, J.B. LIPPINCOTT, PHILADELPHIA, PA, US, vol. 26, no. 4, 1 July 2008 (2008-07-01), pages 326 - 333, XP023610827, ISSN: 0738-081X, [retrieved on 20080805], DOI: 10.1016/J.CLINDERMATOL.2008.01.002
- U. SYDLIK ET AL: "Recovery of neutrophil apoptosis by ectoine: a new strategy against lung inflammation", EUROPEAN RESPIRATORY JOURNAL, vol. 41, no. 2, 25 October 2012 (2012-10-25), pages 433 - 442, XP055186344, ISSN: 0903-1936, DOI: 10.1183/09031936.00132211
- WILLIAM MACNEE: "Accelerated lung aging: a novel pathogenic mechanism of chronic obstructive pulmonary disease (COPD)", BIOCHEMICAL SOCIETY TRANSACTIONS, vol. 37, no. 4, 1 August 2009 (2009-08-01), GB, pages 819 - 823, XP055373857, ISSN: 0300-5127, DOI: 10.1042/BST0370819
- GUARENTE L: "Sirtuins in aging and disease", COLD SPRING HARBOR SYMPOSIA ON QUANTITATIVE BIOLOGY, BIOLOGICAL LABORATORY, COLD SPRING HARBOR, NY, US, vol. 72, 1 January 2007 (2007-01-01), pages 483 - 488, XP009173854, ISSN: 0091-7451, DOI: 10.1101/SQB.2007.72.024
- MOSTOSLAVSKY RAUL ET AL: "Genomic instability and aging-like phenotype in the absence of mammalian SIRT6", CELL, CELL PRESS, US, vol. 124, no. 2, 1 January 2006 (2006-01-01), pages 315 - 329, XP002430108, ISSN: 0092-8674, DOI: 10.1016/J.CELL.2005.11.044
- SHEIKHPOUR: "Antiproliferative effects of Ectoine and Hydroxyectoine on human lung cancer cells", 1 February 2014 (2014-02-01), XP055394921, Retrieved from the Internet <URL:https://www.researchgate.net/profile/Mojhgan_Sheikhpoor/publication/296700982_kngrh_kysh/links/56d949a608aee73df6ce2cf8.pdf> [retrieved on 20170731]
- Z. LIN ET AL: "The Roles of SIRT1 in Cancer", GENES AND CANCER, vol. 4, no. 3-4, 29 January 2013 (2013-01-29), US, pages 97 - 104, XP055395132, ISSN: 1947-6019, DOI: 10.1177/1947601912475079
- V. B. PILLAI ET AL: "Regulation of Akt Signaling by Sirtuins: Its Implication in Cardiac Hypertrophy and Aging", CIRCULATION RESEARCH., vol. 114, no. 2, 16 January 2014 (2014-01-16), US, pages 368 - 378, XP055395157, ISSN: 0009-7330, DOI: 10.1161/CIRCRESAHA.113.300536

## Beschreibung

Die Erfindung betrifft eine Zusammensetzung zur Förderung der Aktivität von Sirtuinen, insbesondere zur Behandlung und/oder Prophylaxe von Erkrankungen, die mit einer verringerten Aktivität von Sirtuinen in Zusammenhang stehen. Hierbei handelt es sich insbesondere um Alterungserscheinungen sowie um Erkrankungen, die mit einer gestörten Zelldifferenzierung im Zusammenhang stehen.

Sirtuine sind zumeist Histon-Deacetylasen des Typs III, die evolutionär hoch konserviert in nahezu allen Lebensformen vorkommen. Der Name leitet sich von "silent Information regulator" ab. Sirtuine deacetylieren Lysin-Reste, insbesondere von Histon-Proteinen am N-terminalen Ende, die als Bestandteil des Chromatins für die Verpackung der DNA verantwortlich sind, wobei als Cofaktor für die Deacetylierung NAD⁺ benötigt wird. Es entstehen somit nach Abspaltung der Acetylgruppe neben dem deacetylierten Protein Nikotinamid und O-Acetyl-ADP-Ribose. Weil das Lysin nach Deacetylierung eine positive Ladung trägt, erhöht sich die Affinität des Histons zum negativ geladenen Rückgrat der DNA. Die DNA wird somit stärker blockiert und die Transkription heruntergeregelt. Sirtuine haben daher einen unmittelbaren Einfluss auf zelluläre Prozesse, da sie in der Lage sind, die Expression bestimmter Proteine und Enzyme zu regulieren. Insgesamt unterscheidet man bei Menschen und anderen Säugetieren 7 verschiedene Sirtuine, SIRT1 bis SIRT7. Neben Histonen werden beispielsweise auch intrazelluläre Signalmoleküle von Sirtuinen deacetyliert.

Aufgrund des Einflusses der Sirtuine auf die Expression von Proteinen und Enzymen, die teilweise in Zusammenhang mit bestimmten Erkrankungen stehen, wird seit einigen Jahren erheblicher Forschungsaufwand in Bezug auf diese Enzymklasse betrieben. Insbesondere scheinen Sirtuine einen positiven Effekt auf verschiedene Alterungserscheinungen bzw. Krankheiten zu haben, die verstärkt im Alter auftreten, beispielsweise weil sie mit einer veränderten Genregulation bei der Zelldifferenzierung in Zusammenhang stehen. Zum Teil wurde daher in der Presse bereits von "Anti-Aging-Enzymen" gesprochen. Unter anderem wird untersucht, wie sich Sirtuin-Aktivatoren, beispielsweise Resveratrol, auswirken.

Zu den Krankheiten, auf die Sirtuine einen Einfluss haben, zählen die bereits erwähnten Alterungserscheinungen, insbesondere solche der Lunge wie das Auftreten eines senilen Emphysems, welches auch als Altersemphysem oder primär atrophisches Emphysem bezeichnet wird. Neben der Alterung der Lunge wird in der Literatur aber auch der Zusammenhang zwischen Sirtuinen und anderen Erkrankungen diskutiert, beispielsweise Herzhypertrophie, Gicht, Atherosklerose bzw. Arteriosklerose, Adipositas, Amyotrophe Lateralsklerose oder Krebserkrankungen, insbesondere aus dem Bereich der Hämatologie wie akute oder chronische Leukämie oder maligne Lymphome. Die Regulierung der Genaktivität über die Histone spielt bei der Zelldifferenzierung eine bedeutende Rolle. Es wurde ein Zusammenhang zwischen einem alterungs- oder krankheitsbedingten Verlust der Sirtuinaktivität und dem Auftreten von Alterungserscheinungen postuliert.

Mojhgan Sheikhpour et al., J Cancer Sci Ther 2014, 6:10 (doi.org/10.4172/1948-5956.) offenbart eine Studie, in der die antiproliferative Wirkung von zwei kompatiblen Lösungsmitteln, Ectoin und 5-Hydroxy-Ectoin, auf eine menschliche Lungenkrebszelllinie (QU-DB) untersucht wurde. Darin wird für Ectoin und Hydroxyectoin ein hemmender und zytotoxischer Effekt auf die Krebszelllinie beschrieben.

Es stellt sich somit die Aufgabe, Substanzen oder Zusammensetzungen zur Verfügung zu stellen, die dem alterungsbedingten Verlust der Sirtuinaktivität entgegenwirken.

Überraschend wurde gefunden, dass Ectoin, Hydroxyectoin sowie Salze, Ester und Amide von Ectoin/Hydroxyectoin in der Lage sind, eine signifikante Verringerung des Verlustes der Sirtuinaktivität herbeizuführen. Die Sirtuinaktivität bezieht sich in erster Linie auf Anzahl, Struktur und/oder Funktion der Sirtuine, d. h. eine verringerte Sirtuinaktivität kann dann vorliegen, wenn Sirtuine weniger aktiv sind oder auch wenn weniger Sirtuine vorhanden sind. Auf diese Weise kann sowohl eine therapeutische Behandlung vorgenommen als auch eine präventive Wirkung erzielt werden.

Bei Ectoin und Hydroxyectoin handelt es sich um Tetrahydropyrimidinderivate, die unter Stressbedingungen von extremophilen, insbesondere halophilen Mikroorganismen synthetisiert werden. Für Ectoin und Hydroxyectoin wurden bislang verschiedene Verwendungen beschrieben, beispielsweise als Moisturizer, zur Behandlung des Vascular Leak Syndroms (VLS) (DE 10 2006 056 766 A1) oder zur Behandlung von Neurodermitis (DE 103 30 243 A1).

Die systematische Bezeichnung für Ectoin lautet 2-Methyl-1,4,5,6-tetrahydropyrimidin-4-carbonsäure, für Hydroxyectoin 5-Hydroxy-2-methyl-1 ,4,5,6-tetrahydropyrimidin-4-carbonsäure.

Die Struktur des natürlichen L-Ectoins ((S)-2-Methyl-1 ,4,5,6-tetrahydropyrimidin-4-carbonsäure) ist im Folgenden dargestellt:

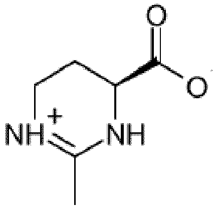

Die Struktur des natürlichen Hydroxyectoins ((4S,5S)-5-Hydroxy-2-methyl-1 ,4,5,6-tetrahydropyrimidin-4-carbonsäure) wird im Folgenden wiedergegeben:

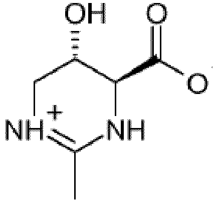

Die Verwendung der angegebenen Stereoisomere ist bevorzugt, jedoch nicht obligatorisch, d.h. auch die Verwendung anderer Stereoisomere bzw. des Racemats ist möglich.

Die erfindungsgemäße Zusammensetzung kann allgemein zur Behandlung und/oder Prophylaxe von Alterungserscheinungen und zur Beeinflussung von Selbsterneuerungsvorgängen (Self-renewal), Zelldifferenzierungen und Zellvermehrungen eingesetzt werden. Besondere Bedeutung hat die Zusammensetzung bei der Behandlung von Alterungserscheinungen der Lunge, insbesondere des senilen Emphysems. Dieses ist gekennzeichnet durch die Degeneration der alveolären Strukturen in der Lunge, die zu einer Verringerung der Lungenoberfläche führt, welche für den Gasaustausch zur Verfügung steht. Der Patient bekommt entsprechend "schlechter Luft". Es konnte gezeigt werden, dass die mehrmalige Applikation einer Ectoin-haltigen Lösung in die Lunge bei Ratten zu einer signifikanten Verringerung des Verlustes an Sirtuinaktivität im Lungengewebe führt. Die Behandlung von auf Schwebstaubeinwirkung beruhenden Lungenerkrankungen wurde zwar bereits in der WO 2005/002556 A1 beschrieben, überraschend wurde jedoch gefunden, dass auch allgemeine Lungenalterungserscheinungen mit Ectoin/Hydroxyectoin zu behandeln sind. Die Alterung von Gewebe beruht auf geänderter Genaktivität bei der Selbsterneuerung und Zelldifferenzierung im (Lungen)gewebe, die über die Zugänglichkeit der DNA durch Histone geregelt und damit auch abhängig von Sirtuinen ist.

Entsprechend ausgenommen von der vorliegenden Erfindung kann die Behandlung/Prophylaxe von Lungenerkrankungen sein, die auf Schwebstaubeinwirkung zurückgeht, wie der WO 2005/002556 A1 entnehmbar. Ebenso ausgenommen sein kann die Behandlung/Prophylaxe von Lungenentzündung, Asthma, COPD, ARDS (Acute Respiratory Distress Syndrome), zystischer Fibrose, Lungenfibrose, Silikose, Sarkoidose, Allergien und bronchialer Hyperreagibilität, die sich aus der WO 2013/034299 A1 ergibt.

Eine weitere Gruppe von Lungenerkrankungen, die sich mit der erfindungsgemäßen Zusammensetzung behandeln lassen bzw. denen mit dieser vorgebeugt werden kann, stellt die Gruppe der idiopathischen interstitiellen Pneumonien (IIP) dar. Die Ursache dieser Erkrankungen ist nicht bekannt, weshalb sie als idiopathisch bezeichnet werden. Sie betreffen vor allem das Bindegewebe der Lunge. Die Patienten leiden unter Atemnot, die im Anfangsstadium auf körperliche Belastungen beschränkt sein kann, sich mit der Zeit jedoch häufig verschlimmert und im Endstadium in respiratorische Insuffizienz übergeht. Es kann zu einem Sauerstoffmangel im Blut kommen. Auch diese Erkrankungen stehen häufig mit einer gestörten Zellvermehrung in Zusammenhang.

Gemäß aktueller Klassifikation unterscheidet man sieben verschiedene Krankheitsformen:
- Idiopathische pulmonale Fibrose (IPF)
- Nicht spezifische interstitielle Pneumonie (NSIP)
- Kryptogene organisierende Pneumonie (COP)
- Akute interstitielle Pneumonie (AIP)
- Respiratorische Bronchiolitis mit interstitieller Lungenerkrankung (RB-ILD)
- Desquamative interstitielle Pneumonie (DIP) und
- Lymphoide interstitielle Pneumonie (LIP).

Hinzu kommen nicht klassifizierbare IIPs für nicht klar abgrenzbare Erkrankungen.

Besonders häufig tritt die idiopathische pulmonale Fibrose (IPF) auf. Die Behandlung mit der erfindungsgemäßen Zusammensetzung spielt hier auch deshalb eine besondere Rolle, weil die Erkrankung sich durch entzündungshemmende Mittel kaum bekämpfen lässt. Gleiches gilt für die akute interstitielle Pneumonie (AIP). Beide Erkrankungen haben normalerweise eine schlechte Prognose, weshalb die Behandlung dieser Erkrankungen erfindungsgemäß eine besondere Rolle spielt.

Zu den Alterungserscheinungen, die sich durch die erfindungsgemäße Zusammensetzung behandeln oder vorbeugen lassen, gehören auch intrinsische Hautalterungserscheinungen. Hierunter werden Alterungserscheinungen der Haut verstanden, die nicht auf externe Einflüsse wie UV-Strahlung oder allgemein Sonneneinstrahlung zurückzuführen sind. Sie haben ihre Ursache in einer verminderten Reagibilität der Hautzellen, ist genetisch bedingt und beginnt in der Regel spätestens mit dem 30. Lebensjahr. Die Haut verliert an Elastizität und Spannkraft. Aufgrund der verminderten Zellteilung der Haut im fortgeschrittenen Alter nimmt der Gehalt an Kollagen und Elastin in der Haut deutlich ab.

Auch Hautalterungserscheinungen, die auf die Einwirkung von Schwebstaub zurückzuführen sind, können durch die erfindungsgemäße Zusammensetzung behandelt oder vorgebeugt werden. Insbesondere Schwebstaub (auch Feinstaub genannt) mit einer mittleren Partikelgröße ≤ 15 µm, besonders ≤ 10 µm kann Hautalterungserscheinungen hervorrufen. Solche Schwebstäube entstehen vielfach durch die Verbrennung fossiler Brennstoffe, aber auch in Form von Sand, Sporen, Pollen, Gesteinstaub, in der Landwirtschaft, im Bergbau, durch Tabakgenuss, Reifenabrieb, Bremsenabrieb oder durch Waldbrände.

Allgemein umfasst die Erfindung insbesondere die Behandlung/Prophylaxe von Alterungserscheinungen und auf gestörte Zelldifferenzierung oder Zellvermehrung zurückzuführende Erkrankungen, die durch endogene Faktoren intrazellulär beeinflusst oder hervorgerufen werden. Die Stärkung der Expression von Sirtuinen durch Ectoin/Hydroxyectoin verbessert die Zelldifferenzierung des Gewebes.

Alle humanen Epithelien und Endothelien, die in Kontakt mit Ectoin/Hydroxyectoin kommen, werden in ihrer Selbsterneuerung und Zelldifferenzierung positiv beeinflusst. Entsprechend betrifft die Erfindung eine Zusammensetzung zur Behandlung von endothelialen, epithelialen und neurologischen Erkrankungen. Neben den bereits oben genannten Erkrankungen existieren weitere Erkrankungen, die als Alterungserscheinungen in dem Sinne zu bezeichnen sind, dass sie beim Menschen bevorzugt in fortgeschrittenem Alter auftreten, bzw. die mit einer gestörten Zelldifferenzierung/Zellvermehrung in Zusammenhang stehen. Hierzu gehören: Herzhypertrophie, Gicht, Arteriosklerose/Atherosklerose, Adipositas, Amyotrophe Lateralsklerose (ALS) und Krebserkrankungen. Bei den Krebserkrankungen handelt es sich insbesondere um solche aus dem Bereich der Hämatologie wie akute oder chronische Leukämie und maligne Lymphome. Ebenfalls behandelbar sind epitheliale und mesenchymale Tumoren, d. h. allgemein Karzinome und Sarkome. Als behandelbare Krebserkrankungen sind darüber hinaus zu nennen: Lungenkrebs, Hautkrebs, Darmkrebs (insbesondere Dickdarmkrebs), Brustkrebs, Prostatakrebs, Bauchspeicheldrüsenkrebs, Speiseröhrenkrebs, Kehlkopfkrebs, Magenkrebs, Gebärmutterhalskrebs, Eierstockkrebs, Hodenkrebs, Nierenkrebs, Harnblasenkrebs, Schilddrüsenkrebs, Mundhöhlenkrebs, Rachenkrebs und Hirntumore.

Weitere behandelbare Erkrankungen aus dem Bereich der Hämatologie sind Anämie oder MDS (Myelodysplastisches Syndrom). Auch zur Behandlung und Prophylaxe dieser Erkrankungen ist Ectoin/Hydroxyectoin geeignet. Die Therapie der Amyotrophen Lateralsklerose ist darauf zurückzuführen, dass Sirtuine einen stimulierenden Effekt auf das Wachstum von Neuronen und Axonen zeigen.

Bei dem Sirtuin, dessen Aktivität erhöht bzw. dessen Aktivitätsverlust verringert wird, handelt es sich insbesondere um SIRT-1, welches u. a. kardioprotektiv, Krebs hemmend, antiinflammatorisch und antioxidativ wirkt. Von besonderer Bedeutung ist daneben SIRT6. Es konnte gezeigt werden, dass ein Mangel dieser Sirtuine bei Mäusen zu vorzeitiger Alterung führt (Cheng et al., Proc Natl. Acad Sci USA 2003, 100, 10794-10799; Mostoslavsky et al., Cell 2006, 124, 315-329).

Als pharmakologisch verträgliche Salze des Ectoins/Hydroxyectoins kommen die Alkali- oder Erdalkalisalze, insbesondere die Salze des Kaliums, Natriums, Magnesiums und Calciums, aber auch Salze mit organischen Basen wie z. B. mit nicht toxischen aliphatischen oder aromatischen Aminen in Frage.

Durch Umsetzung der Carboxylgruppe des Ectoins/Hydroxyectoins mit Alkoholen oder Aminen, können entsprechende Ester oder Amide erhalten werden, die ebenfalls erfindungsgemäß einsetzbar sind. In diesem Fall ist die COOH-Gruppe des Ectoins/Hydroxyectoins durch eine Carbonsäureesterfunktion COOR bzw. eine Carbonsäureamidfunktion CONHR' oder CONR'R" ersetzt, wobei R, R', R" unabhängig voneinander für gesättigte oder ungesättigte, geradkettige oder verzweigte Alkyl-, Cycloalkyl-, Aryl-, Heteroaryl-, Alkylaryl-, Arylalkyl-, Alkoxyalkyl-, Alkylthioalkyl-, Aryloxyalkyl- oder Arylthioalkylgruppen stehen. Insbesondere kann es sich um eine C₁₋, C₂₋, C₃₋, C₄₋, C₅₋, C6₋, C₇₋, C₈₋, C₉₋, C₁₀₋, C₁₁₋, C₁₂₋, C₁₃₋, C₁₄₋, C₁₅₋, C₁₆₋, C₁₇₋ oder C₁₈₋ Alkylgruppe handeln. Beim Hydroxyectoin kann auch die Hydroxygruppe mit einer Carbonsäure unterschiedlicher Kettenlänge zu einem entsprechenden Ester umgesetzt sein. Auch die jeweiligen Ester oder Amide können in ionischer oder zwitterionischer Form vorliegen, d.h. die Erfindung schließt die Verwendung von Salzen der genannten Ester oder Amide ein.

Auch die Verwendung des Ectoinamids der 2-Hydroxy-5-aminobenzoesäure ist möglich. Die Strukturformel ist im Folgenden wiedergegeben:

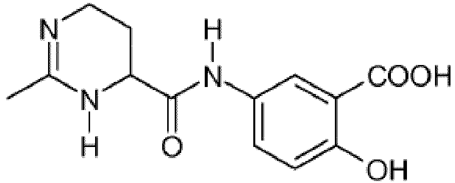

Es handelt sich somit um das 2-Methyl-1 ,4,5,6-tetrahydropyrimidin-4-carbonsäureamid der 2-Hydroxy-5-aminobenzoesäure. Bevorzugt handelt es sich um das entsprechende Amid des L-Ectoins: (S)-2-Methyl-1,4,5,6-tetrahydropyrimidin-4-carbonsäureamid. Denkbar ist auch die Verwendung des Hydroxyectoinamids der 2-Hydroxy-5-aminobenzoesäure.

Die Zusammensetzung kann übliche Hilfsstoffe enthalten, z. B. Trägermittel, Konservierungsmittel, Bakterizide, Lösungsvermittler, Vitamine, Stabilisatoren, Substanzen zum Verhindern des Schäumens, osmotisch aktive Substanzen, Farbstoffe, oberflächenaktive Substanzen, Emulgatoren, feuchthaltende Substanzen, Viskositätserhöhende Mittel u. ä.. Im Fall der Verabreichung in fester Form, bspw. mittels Pulverinhalatoren, ist es sinnvoll, nur leicht resorbierbare nichtreizende Trägerstoffe wie mikronisierte Lactose einzusetzen.

Konservierungsmittel sind beispielsweise Thiomersal, organische Quecksilberverbindungen wie Phenylquecksilber, Benzalkoniumchlorid, Chlorhexidin, Benzylalkohol, Glucose, Ethanol und quartäre Ammoniumsalze.

Die Formulierungen der Erfindung können ebenfalls geeignete Puffersysteme oder andere Hilfsstoffe zur pH-Einstellung beinhalten, um einen gewünschten pH-Wert einzustellen und aufrechtzuerhalten. Typischerweise handelt es sich um pH-Werte zwischen 4 und 8, vorzugsweise zwischen 5 und 7,5, weiter bevorzugt ca. 7. Geeignete Puffersysteme sind Citrat, Phosphat, TRIS, Glycin, Borat, Acetat. Diese Puffersysteme können hergestellt werden aus Substanzen wie Zitronensäure, Mononatriumphosphat, Dinatriumphosphat, Glycin, Borsäure, Natriumtetraborat, Essigsäure oder Natriumacetat.

Die Zusammensetzungen können weitere Wirkstoffe enthalten, es ist jedoch ohne weiteres möglich und für die Behandlung oder Vorbeugung der Alterungserscheinungen ausreichend, Zusammensetzungen zu verwenden, die lediglich Ectoin und/oder Hydroxyectoin bzw. entsprechende Salze, Ester oder Amide als Wirkstoff enthalten.

Die Konzentration an Ectoin/Hydroxyectoin und/oder entsprechenden Salzen, Estern oder Amiden kann insbesondere in einem Bereich von 10 bis 500 mM, bevorzugt 50 bis 500 mM, besonders bevorzugt 100 bis 500 mM bzw. 100 bis 200 mM liegen. Diese Konzentrationen haben sich als geeignet zur Herbeiführung des erfindungsgemäßen Effekts erwiesen. Insbesondere kann der Anteil an Ectoin/Hydroxyectoin und/oder entsprechenden Salzen, Estern oder Amiden in der Zusammensetzung in einem Bereich von 0,05 bis 20 Gew.- %, bevorzugt 0,1 bis 10 Gew.-% liegen. Eine gute Wirkung konnte beispielsweise in einem Bereich zwischen 0,5 und 2 Gew.-% beobachtet werden.

Die Zusammensetzung kann als Lösung, bevorzugt als wässrige Lösung vorliegen. Ebenso möglich ist das Vorliegen als Suspension, Emulsion oder Mikroemulsion.

Um die Applikation und die Haltbarkeit der erfindungsgemäßen Zusammensetzung zu verbessern, kann die den Wirkstoff enthaltende Zusammensetzung auch in Nanostrukturen eingekapselt sein oder in Form von Liposomen verabreicht werden. Dies ist insbesondere dann vorteilhaft, wenn die Zusammensetzung kein Konservierungsmittel enthält. Entsprechende Verfahren zur Verkapselung und zur Herstellung von Liposomen sind grundsätzlich aus dem Stand der Technik bekannt.

Des Weiteren wird eine Sprühvorrichtung offenbart, mit der die erfindungsgemäße Zusammensetzung inhaliert werden kann. Die Sprühvorrichtung weist Mittel zur Zerstäubung der Zusammensetzung auf und verfügt über eine Austrittsöffnung, die dem Benutzer die Inhalation ermöglicht. Eine Sprühvorrichtung ist insbesondere im Zusammenhang mit der Behandlung oder Vorbeugung von Alterungserscheinungen der Lunge von Bedeutung, insbesondere des senilen Emphysems. Geeignete Sprühvorrichtungen können der Inhalation eines Aerosols oder eines Pulvers dienen. Als Pulverinhalatoren sind beispielsweise Spinhaler, Diskhaler, Turbohaler, Rotahaler oder Aerolizer verwendbar.

## Patentansprüche

1. Zusammensetzung, enthaltend als Wirkstoff Ectoin, Hydroxyectoin und/oder Salze, Ester oder Amide dieser Verbindungen zur Verwendung in einem Verfahren zur Behandlung und/oder Prophylaxe von Alterungserscheinungen der Lunge, ausgenommen COPD, und/oder einer idiopathischen interstitiellen Pneumonie (IIP), wobei die Alterungserscheinung der Lunge ein seniles Emphysem ist.

2. Zusammensetzung zur Verwendung nach Anspruch 1, **dadurch gekennzeichnet, dass** die idiopathische interstitielle Pneumonie eine idiopathische pulmonale Fibrose (IPF) oder eine akute interstitielle Pneumonie (AIP) ist.

3. Zusammensetzung zur Verwendung nach einem der Ansprüche 1 bis 2, **dadurch gekennzeichnet, dass** eine Verringerung des Verlustes der Sirtuinaktivität herbeigeführt wird.

4. Zusammensetzung zur Verwendung nach einem der Anspruche 1 bis 3, **dadurch gekennzeichnet, dass** die Zusammensetzung als Lösung, Suspension, Emulsion oder Mikroemulsion vorliegt.

5. Zusammensetzung zur Verwendung nach Anspruch 4, **dadurch gekennzeichnet, dass** die Lösung eine wässrige Lösung ist.

6. Zusammensetzung zur Verwendung nach einem der Anspruche 1 bis 5 zur Applikation durch Inhalation.

7. Zusammensetzung zur Verwendung nach einem der Anspruche 1 bis 6, **dadurch gekennzeichnet, dass** die Konzentration an Ectoin, Hydroxyectoin und/oder Salzen, Estern oder Amiden dieser Verbindungen in der Zusammensetzung 10 bis 500 mM, bevorzugt 100 bis 500 mM beträgt.

8. Zusammensetzung zur Verwendung nach einem der Anspruche 1 bis 7, **dadurch gekennzeichnet, dass** die Zusammensetzung in Nanostrukturen eingekapselt ist oder in Form von Liposomen vorliegt.

## Claims

1. Composition comprising as active ingredient ectoine, hydroxyectoine and/or salts, esters or amides of these compounds for use in a method of treatment and/or prophylaxis of lung ageing symptoms, excluding COPD, and/or idiopathic interstitial pneumonia (IIP), wherein the lung ageing symptom is senile emphysema.

2. Composition for use according to claim 1, **characterized in that** the idiopathic interstitial pneumonia is idiopathic pulmonary fibrosis (IPF) or acute interstitial pneumonia (AIP).

3. Composition for use according to any one of claims 1 to 2, **characterized in that** a reduction in the loss of sirtuin activity is induced.

4. Composition for use according to any one of claims 1 to 3, **characterized in that** the composition is present as a solution, suspension, emulsion or microemulsion.

5. Composition for use according to claim 4, **characterized in that** the solution is an aqueous solution.

6. Composition for use according to any one of claims 1 to 5 for application by inhalation.

7. Composition for use according to one of claims 1 to 6, **characterized in that** the concentration of ectoine, hydroxyectoine and/or salts, esters or amides of these compounds in the composition is 10 to 500 mM, preferably 100 to 500 mM.

8. Composition for use according to one of claims 1 to 7, **characterized in that** the composition is encapsulated in nanostructures or is present in the form of liposomes.

## Revendications

1. Composition contenant, comme principe actif, de l'ectoïne, de l'hydroxyectoïne et/ou des sels, esters ou amides de ces composés, destinée à être utilisée dans un procédé de traitement et/ou de prophylaxie du vieillissement des poumons, à l'exception de la BPCO, et/ou d'une pneumonie interstitielle idiopathique (PII), dans laquelle le vieillissement des poumons est un emphysème sénile.

2. Composition à utiliser selon la revendication 1, **caractérisée en ce que** la pneumonie interstitielle idiopathique est une fibrose pulmonaire idiopathique (FPI) ou une pneumonie interstitielle aiguë (PIA).

3. Composition à utiliser selon l'une des revendications 1 à 2, **caractérisée en ce qu'**une réduction de la perte d'activité de la sirtuine est provoquée.

4. Composition à utiliser selon l'une des revendications 1 à 3, **caractérisée en ce que** la composition se présente sous la forme d'une solution, d'une suspension, d'une émulsion ou d'une microémulsion.

5. Composition à utiliser selon la revendication 4, **caractérisée en ce que** la solution est une solution aqueuse.

6. Composition à utiliser selon l'une des revendications 1 à 5 pour l'application par inhalation.

7. Composition à utiliser selon l'une des revendications 1 à 6, **caractérisée en ce que** la concentration en ectoïne, en hydroxyectoïne et/ou en sels, esters ou amides de ces composés dans la composition est de 10 à 500 mM, de préférence de 100 à 500 mM.

8. Composition à utiliser selon l'une des revendications 1 à 7, **caractérisée en ce que** la composition est encapsulée dans des nanostructures ou se présente sous forme de liposomes.
